# EUROPEAN PATENT SPECIFICATION

(11) **EP 2 226 047 B1**
(45) Date of publication and mention of the grant of the patent: **26.06.2019**
(21) Application number: 09154506.1
(22) Date of filing: 06.03.2009
(51) Int. Cl.: A61F 13/533, A61F 13/536

(54) **ABSORBENT CORE**
SAUGFÄHIGER KERN
NOYAU ABSORBANT

(43) Date of publication of application: 08.09.2010
(73) Proprietor: The Procter & Gamble Company, Cincinnati, OH 45202 (US)
(72) Inventor: Carlucci, Giovanni, 66100, Chieti (Italy) (IT); Tamburro, Maurizio, 66020, Sambuceto (Chieti) (IT)
(74) Representative: Kremer, Véronique Marie Joséphine

(56) References cited:
- WO-A-96/19173
- WO-A-03/068121
- WO-A-2006/039188
- GB-A- 2 283 680
- US-A- 5 118 376
- US-A1- 2003 028 165

## Description

### FIELD OF THE INVENTION

The present invention relates to an absorbent core for absorbent articles, for example sanitary napkins and the like.

### BACKGROUND OF THE INVENTION

Absorbent articles for absorption of body fluids such as menses or blood or vaginal discharges are well known in the art, and comprise for example feminine hygiene articles such as sanitary napkins, panty liners, tampons, interlabial devices, as well as wound dressings, and the like. When considering for example sanitary napkins, these articles typically comprise a liquid-pervious topsheet as wearer-facing layer, a backsheet as garment-facing layer and an absorbent core between topsheet and backsheet. The body fluids are acquired through the topsheet and subsequently stored in the absorbent core. The backsheet typically prevents the absorbed fluids from wetting the wearer's garment.

An absorbent core can typically comprise one or more fibrous absorbent materials, which in turn can comprise natural fibres, such as for example cellulose fibres, typically wood pulp fibres, synthetic fibres, or combinations thereof.

Absorbent articles can further comprise, typically in the absorbent core, superabsorbent materials, such as absorbent gelling materials (AGM), usually in finely dispersed form, e.g. typically in particulate form, in order to improve their absorption and retention characteristics. Superabsorbent materials for use in absorbent articles typically comprise water-insoluble, water-swellable, hydrogel-forming crosslinked absorbent polymers which are capable of absorbing large quantities of liquids and of retaining such absorbed liquids under moderate pressure. Absorbent gelling materials can be incorporated in absorbent articles, typically in the core structure, in different ways; for example, absorbent gelling materials in particulate form can be dispersed among the fibres of fibrous layers comprised in the core, or rather localized in a more concentrated arrangement between fibrous layers.

Absorbent cores for absorbent articles having a thin structure can further provide an improved immobilization of absorbent gelling materials, particularly when the article is fully or partially loaded with liquid, and an increased wearing comfort. Such thinner structures provide absorbent articles combining better comfort, discreetness and adaptability, such as for example, thin absorbent structures where the absorbent gelling material is located and somehow kept in selected, e.g. patterned regions of the structure itself.

EP 1447067, assigned to the Procter & Gamble Company, describes an absorbent article, typically a disposable absorbent article, such as a diaper, having an absorbent core which imparts increased wearing comfort to the article and makes it thin and dry. The absorbent core comprises a substrate layer, the substrate layer comprising a first surface and a second surface, the absorbent core further comprising a discontinuous layer of absorbent material, the absorbent material comprising an absorbent polymer material, the absorbent material optionally comprising an absorbent fibrous material which does not represent more than 20 weight percent of the total weight of the absorbent polymer material. The discontinuous layer of absorbent material comprises a first surface and a second surface, the absorbent core further comprising a layer of thermoplastic material, the layer of thermoplastic material comprising a first surface and a second surface and wherein the second surface of the discontinuous layer of absorbent material is in at least partial contact with the first surface of the substrate layer and wherein portions of the second surface of the layer of thermoplastic material are in direct contact with the first surface of the substrate layer and portions of the second surface of the layer of thermoplastic material are in direct contact with the first surface of the discontinuous layer of absorbent material.

While absorbent articles according to EP 1447067 and comprising thin absorbent cores with relatively high amounts of absorbent gelling materials and rather low content of fibrous materials commonly have good absorption and retention characteristics to body fluids, they can be still improved to achieve faster absorbency, reduced tendency to bunching and roping, particularly in the crotch region, without substantially reducing either the comfort of the article in use, or its overall absorbency. Absorbent articles comprising such thin absorbent cores can in fact be subject to deformation when worn under the pressure and forces exerted by the wearer's body and garment during use. Typically, an absorbent article such as a sanitary napkin can be compressed in transverse direction, i.e. in a direction generally perpendicular to its major, longitudinal axis, under the forces exerted by the wearer's thighs, and can deform, particularly in the crotch region, for example usually along longitudinal fold lines. Upon relief of said forces during wear a thin article can have a reduced capacity of recovering its initial configuration, particularly in a wet state after liquid absorption, and thus can lead to a less than optimal fit to the body anatomy. This can in turn reduce the area of the article available for fluid acquisition, owing to the folds and creases, and translate for the article into a reduced comfort, and in a decreased utilization of the core absorption capacity over the wearing time.

Other absorbent cores are disclosed in US5118376,US2003/0018265, GB2283680, WO2006/039188, WO96/19173, WO03/068121.

The present invention provides significant improvements in the above area by the incorporation of a layer of foam material in an absorbent core structure for an absorbent article, which comprises the absorbent gelling material in a layer stably provided onto a substrate layer.

### SUMMARY OF THE INVENTION

The present invention addresses the above needs by providing an absorbent core according to claim 1 for an absorbent article intended for absorption of menses or blood or vaginal discharges.

### BRIEF DESCRIPTION OF THE DRAWINGS

Figure 1 is a plan view of a sanitary napkin showing an absorbent core according to an embodiment of the present invention, with portions of some constituent elements cut out in order to show underlying elements.
Figure 2 is a schematic cross section of the sanitary napkin of Figure 1 taken in the transverse axis A-A'.
Figure 3 shows a schematic cross section of an absorbent core according to one embodiment of the present invention.
Figure 4 shows a schematic cross section of an absorbent core according to another embodiment of the present invention.
Figure 5 shows a perspective view of an exemplary absorbent core according to the present invention.

### DETAILED DESCRIPTION OF THE INVENTION

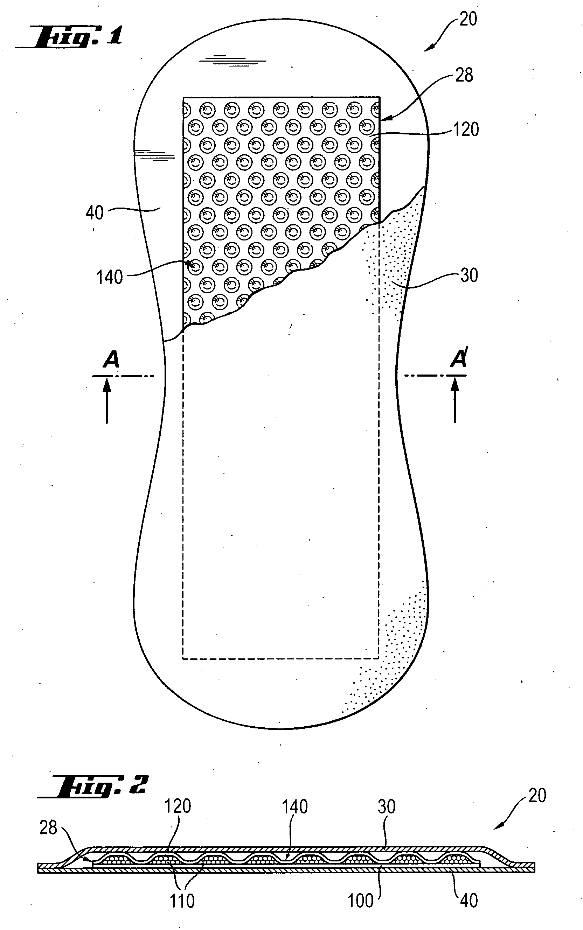

The present invention relates to an absorbent core for absorbent articles such as sanitary napkins, panty liners, tampons, interlabial devices, wound dressings, diapers, adult incontinence articles, and the like, which are intended for the absorption of body fluids, such as menses or blood or vaginal discharges or urine. Exemplary absorbent articles in the context of the present invention are disposable absorbent articles. The term "disposable" is used herein to describe articles, which are not intended to be laundered or otherwise restored or reused as an article (i.e. they are intended to be discarded after a single use and possibly to be recycled, composted or otherwise disposed of in an environmentally compatible manner). The absorbent article comprising an absorbent core according to the present invention can be for example a sanitary napkin or a panty liner. The absorbent core of the present invention will be herein described in the context of a typical absorbent article, such as, for example, a sanitary napkin 20 as illustrated in Figure 1. Typically, such articles as shown in Figure 1 can comprise the elements of a liquid pervious topsheet 30, a backsheet 40 and an absorbent core 28 intermediate said topsheet 30 and said backsheet 40.

In the following description of the invention, the surface of the article, or of each element thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

### Topsheet

According to the present invention, the absorbent article can comprise a liquid pervious topsheet. The topsheet suitable for use herein can comprise wovens, non-wovens, and/or three-dimensional webs of a liquid impermeable polymeric film comprising liquid permeable apertures. In Figure 1 the topsheet is indicated with reference numeral 30. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

### Absorbent Core

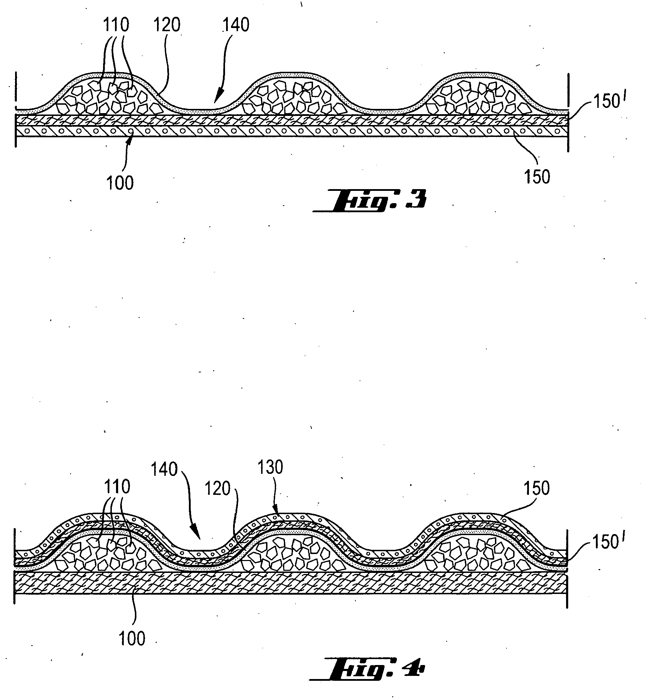

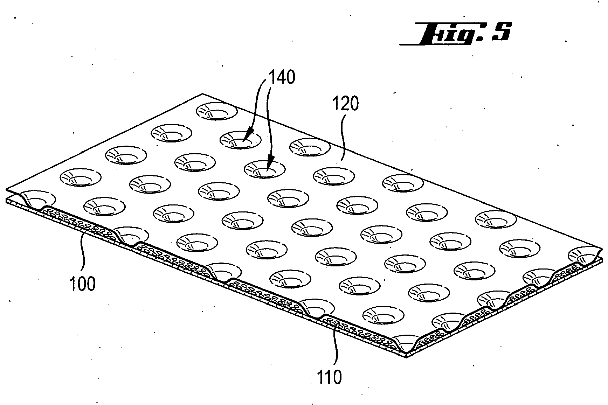

According to an embodiment of the present invention, and as shown for example in the embodiments of Figures 3 and 5, the absorbent core 28 comprises a substrate layer 100, a layer of absorbent polymer material 110, a layer of adhesive 120. Typically the adhesive can be a hot melt adhesive. In an embodiment of the present invention, the layer of adhesive 120 can be typically for example a layer of fiberized hot melt adhesive 120. The substrate layer 100 can for example comprise a fibrous material.

An alternative embodiment of the present invention is shown in Figure 4. The absorbent core shown in Figure 4 can further comprise a cover layer 130. Suitable materials for the cover layer can be for example nonwoven materials.

The substrate layer 100 comprises a first surface and a second surface. Conventionally, in all the sectional views illustrated in the attached drawings the first surface of each layer is meant to correspond to the top surface, in turn, unless stated otherwise, corresponding to the wearer facing surface of the article 20 incorporating the absorbent core, while the second surface corresponds to the bottom surface, hence in turn the garment facing surface. At least portions of the first surface of the substrate layer 100 are in contact with a layer of absorbent polymer material 110. This layer of absorbent polymer material 110 comprises a first surface and a second surface, and can be typically a uniform or non uniform layer, wherein by "uniform" or "non uniform" it is meant that the absorbent polymer material 110 can be distributed over the substrate layer 100 respectively with uniform or non uniform basis weight over the area interested by the distribution. Conversely, the second surface of the layer of absorbent polymer material 110 is in at least partial contact with the first surface of the substrate layer 100. According to an embodiment of the present invention, the layer of absorbent polymer material 110 can also be a discontinuous layer that is a layer typically comprising openings, i.e. areas substantially free of absorbent polymer material, which in certain embodiments can be typically completely surrounded by areas comprising absorbent polymer material. Typically these openings have a diameter or largest span of less than 10 mm, or less than 5 mm, or 3 mm, or 2 mm, or 1.5 mm and of more than 0.5 mm, or 1 mm. At least portions of the second surface of the absorbent polymer material layer 110 are in contact with at least portions of the first surface of the substrate layer material 100. The first surface of the layer of absorbent polymer material 110 defines a certain height of the layer of absorbent polymer material above the first surface of the layer of substrate material 100. When the absorbent polymer material layer 110 is provided as a non uniform layer, typically for example as a discontinuous layer, at least some portions of the first surface of the substrate layer 100 are not covered by absorbent polymer material 110. The absorbent core 28 further comprises a layer of adhesive 120, for example typically a hot melt adhesive. This hot melt adhesive 120 serves to at least partially immobilize the absorbent polymer material 110. According to an embodiment of the present invention, the adhesive 120 can be typically a fiberized hot melt adhesive, i.e., being provided in fibres as a fibrous layer.

In an alternative embodiment of the present invention, as illustrated in Figure 4, the absorbent core 28 can further comprise a cover layer 130 having respective first and second surface, positioned such that the second surface of the cover layer 130 is in contact with the first surface of the layer of typically hot melt adhesive 120.

In an embodiment of the present invention comprising e.g. a non uniform layer of absorbent polymer material 110 the typically hot melt adhesive 120, for example typically provided as a fibrous layer, can be partially in contact with the absorbent polymer material 110 and partially in contact with the substrate layer 100. Figures 3 and 5 show such a structure in an exemplary embodiment of the present invention. In this structure the absorbent polymer material layer 110 is provided as a discontinuous layer, a layer of typically hot melt adhesive 120 is laid down onto the layer of absorbent polymer material 110, typically, for example, in fiberized form, such that the second surface of the hot melt adhesive layer 120 is in direct contact with the first surface of the layer of absorbent polymer material 110, but also in direct contact with the first surface of the substrate layer 100, where the substrate layer is not covered by the absorbent polymer material 110, i.e. typically in correspondence of the openings of the discontinuous layer of the absorbent polymer material 110. By saying "in direct contact", as well as more generally "in contact", as used herein, it is meant that there is no further intermediate component layer between e.g. the layer of hot melt adhesive 120 and the other respective layer in direct contact thereto, such as for example a further fibrous layer. It is however not excluded that a further adhesive material can be comprised between the layer of e.g. hot melt adhesive 120 and the optional cover layer 130, when present, as shown in Figure 4, or the layer of absorbent polymer material 110 or, more typically, the substrate layer 100, such as for example a supplementary adhesive material provided onto the first surface of the substrate layer 100 to further stabilize the overlying absorbent polymer material 110. "In direct contact" and "in contact" can hence be considered to comprise in this context a direct adhesive contact between the layer of hot melt adhesive 120 and another respective layer as explained above, or more in general direct adhesive contact between two layers, e.g. the layer of absorbent polymer material and the substrate layer. This imparts an essentially three-dimensional structure to the fibrous layer of hot melt adhesive 120 which in itself is essentially a two-dimensional structure of relatively small thickness (in z-direction), as compared to the extension in x- and y-direction. In other words, the hot melt adhesive 120 undulates between the first surface of the absorbent polymer material 110 and the first surface of the substrate layer 100. The areas where the hot melt adhesive 120 is in direct contact with the substrate layer 100, when present according an embodiment of the present invention, are the areas of junction 140.

Thereby, in such an embodiment the typically hot melt adhesive 120 can provide spaces to hold the absorbent polymer material 110 typically towards the substrate layer 100, and can thereby immobilize this material. In a further aspect, the hot melt adhesive 120 can bond to the substrate 100 thus affixing the absorbent polymer material 110 to the substrate 100. Typical hot melt adhesive materials can also penetrate into both the absorbent polymer material 110 and the substrate layer 100, thus providing for further immobilization and affixation.

In the alternative embodiment representatively illustrated in Figure 4 portions of the cover layer 130 bond to portions of the substrate layer 100 via the hot melt adhesive 120. Thereby, the substrate layer 100 together with the cover layer 130 can provide spaces to immobilize the absorbent polymer material 110.

Of course, while the typically hot melt adhesive materials disclosed herein can provide a much improved wet immobilisation, i.e. immobilisation of absorbent polymer material when the article is wet or at least partially loaded, these hot melt adhesive materials can also provide a very good immobilisation of absorbent polymer material when the article is dry.

In accordance with an embodiment of the present invention, the absorbent polymer material 110 may also be optionally mixed with fibrous material, which can provide a matrix for further immobilization of the absorbent polymer material. However, typically a relatively low amount of fibrous material can be used, for example less than 40 weight %, less than 20 weight %, or less than 10 weight % of the total weight of the absorbent polymer material 110, positioned within the areas of absorbent polymer material.

According to an embodiment of the present invention, in a typically discontinuous layer of absorbent polymer material 110 the areas of absorbent polymer material can be connected to one another, while the areas of junction 140 can be areas, which in an embodiment may correspond to the openings in the discontinuous layer of absorbent polymer material, as shown for example in Figure 5. The areas of absorbent polymer material are then referred to as connected areas. In an alternative embodiment, the areas of junction 140 can be connected to one another. Then, the absorbent polymer material can be deposited in a discrete pattern, or in other words the absorbent polymer material represents islands in a sea of hot melt adhesive 120. Hence, in summary, a discontinuous layer of absorbent polymer material 110 may comprise connected areas of absorbent polymer material 110, as e.g. illustrated in Figure 5, or may alternatively comprise discrete areas of absorbent polymer material 110.

The present invention, and specifically the embodiments described with reference to Figures 3, 4 and 5 can be typically used to provide the absorbent core of an absorbent article, as illustrated in Figure 1. In that case, no further materials wrapping the core, such as for example a top layer and a bottom layer are being used. With reference to the embodiments of Figure 4 the optional cover layer 130 may provide the function of a top layer and the substrate layer 100 may provide the function of a bottom layer of an absorbent core, wherein top and bottom layers respectively correspond to the body facing and garment facing surfaces of the core 28 in an absorbent article.

With reference to Figures 3, 4 and 5, according to exemplary embodiments of the present invention, the areas of direct contact between the hot melt adhesive 120 and the substrate material 100 are referred to as areas of junction 140. The shape, number and disposition of the areas of junction 140 will influence the immobilization of the absorbent polymer material 110. The areas of junction can be for example of squared, rectangular or circular shape. Areas of junction of circular shape can have a diameter of more than 0.5 mm, or more than 1 mm, and of less than 10 mm, or less than 5 mm, or less than 3 mm, or less than 2 mm, or less than 1.5 mm. If the areas of junction 140 are not of circular shape, they can be of a size as to fit inside a circle of any of the diameters given above.

The areas of junction 140, when present, can be disposed in a regular or irregular pattern. For example, the areas of junction 140 may be disposed along lines as shown in Figure 5. These lines may be aligned with the longitudinal axis of the absorbent core, or alternatively they may have a certain angle in respect to the longitudinal edges of the core. A disposition along lines parallel with the longitudinal edges of the absorbent core 28 might create channels in the longitudinal direction which can lead to a lesser wet immobilization, hence for example the areas of junction 140 can be arranged along lines which form an angle of 20 degrees, or 30 degrees, or 40 degrees, or 45 degrees with the longitudinal edges of the absorbent core 28. Another pattern for the areas of junction 140 can be a pattern comprising polygons, for example pentagons and hexagons or a combination of pentagons and hexagons. Also typical can be irregular patterns of areas of junction 140, which also can give a good wet immobilization. Irregular patterns of areas of junction 140 can also give a better fluid handling behaviour in case of absorption of menses or blood or vaginal discharges, since fluid can start diffusing in whichever direction from any initial acquisition point with substantially the same probability of contacting the absorbent polymer material in the e.g. discontinuous layer. Conversely, regular patterns might create preferential paths the fluid could follow with lesser probability of actually contacting the absorbent polymer material.

According to the present invention the adhesive layer 120 can comprise any suitable adhesive material. Typically, the adhesive layer 120 can comprise any suitable hot melt adhesive material.

Without wishing to be bound by theory it has been found that those hot melt adhesive materials can be most useful for immobilizing the absorbent polymer material 110, which combine good cohesion and good adhesion behaviour. Good adhesion can typically ensure that the hot melt adhesive layer 120 maintains good contact with the absorbent polymer material 110 and in particular with the substrate material 100. Good adhesion is a challenge, namely when a nonwoven substrate material is present. Good cohesion ensures that the adhesive does not break, in particular in response to external forces, and namely in response to strain. The adhesive is subject to external forces when the absorbent product has acquired liquid, which is then stored in the absorbent polymer material 110 which in response swells. An exemplary adhesive should allow for such swelling, without breaking and without imparting too many compressive forces, which would restrain the absorbent polymer material 110 from swelling. It may be desirable that the adhesive does not break, which would deteriorate the wet immobilization. Exemplary suitable hot melt adhesive materials can be as described in the already mentioned patent application EP 1447067, particularly at sections [0050] to [0063].

The adhesive material, typically a hotmelt adhesive material, can be typically present in the form of fibres throughout the core, being provided with known means, i.e. the typically hot melt adhesive can be fiberized. Typically, the fibres can have an average thickness from about 1 µm to about 100 µm, or from about 25 µm to about 75 µm, and an average length from about 5 mm to about 50 cm. In particular the layer of typically hot melt adhesive material can be provided such as to comprise a net-like structure.

To improve the adhesiveness of the typically hot melt adhesive material 120 to the substrate layer 100 or to any other layer, in particular any other non-woven layer, such layers may be pre-treated with an auxiliary adhesive.

In particular, typical parameters of a hot melt adhesive in accordance with the present invention can be as follows.

In an aspect, the loss angle tan Delta of the adhesive at 60°C should be below the value of 1, or below the value of 0.5. The loss angle tan Delta at 60°C is correlated with the liquid character of an adhesive at elevated ambient temperatures. The lower tan Delta, the more an adhesive behaves like a solid rather than a liquid, i.e. the lower its tendency to flow or to migrate and the lower the tendency of an adhesive superstructure as described herein to deteriorate or even to collapse over time. This value is hence particularly important if the absorbent article is used in a hot climate.

In a further aspect, typical hot melt adhesives in accordance with the present invention may have a sufficient cohesive strength parameter γ. The cohesive strength parameter γ is measured using the Rheological Creep Test as referred to hereinafter. A sufficiently low cohesive strength parameter γ is representative of elastic adhesive which, for example, can be stretched without tearing. If a stress of τ = 1000 Pa is applied, the cohesive strength parameter γ can be less than 100%, less than 90%, or less than 75%. For a stress of τ = 125000 Pa, the cohesive strength parameter γ can be less than 1200%, less than 1000%, or less than 800%.

According to the present invention, the absorbent core comprises at least one layer of a foam material 150. Foam materials which can be used in the present invention are known and can typically comprise polyurethanes such as those disclosed e.g. in US 6,852,905. According to an embodiment of the present invention, the foam material can be typically water insoluble. Open or closed cell foam materials can be used in the present invention. Suitable foam materials according to the present invention can be typically hydrophilic, although not necessarily water absorbent per se; water absorbency of course can also be a characteristic of foam materials which can be used in the absorbent core of the present invention. Examples of foam materials according to the present invention can comprise polyurethane foams, or melamine foams, such as the hydrophilic open-cell resilient foams containing melamine-formaldehyde disclosed in US 6,800,666, useful in hygiene articles to acquire, distribute and immobilize body fluids.

An example of absorbent foam materials that can be used in the present invention include products that result from the polymerization of certain water-in-oil emulsions having therein a relatively high ratio of water phase to oil phase. Emulsions of this type, which have these relatively high water to oil phase ratios are known in the art as high internal phase emulsions ("HIPEs" or "HIPE" emulsions). The polymeric foam materials that result from the polymerization of such emulsions are referred to herein as "HIPE foams." Examples of HIPE foams are found in U.S. Pat. No's 5,260,345; 5,387,207; 5,817,704; 5,550,167; 5,827,909; 6,365,642; 6,369,121; 6,525,106; 6,362,244.

The relative amounts of the water and oil phases used to form the polymeric precursor HIPE foam emulsions are, among many other parameters, important in determining the structural, mechanical and performance properties of the resulting HIPE foams. In particular, the ratio of water to oil in the foam-forming emulsion can influence foam density, cell size, specific surface area of the foam and dimensions of the struts that form the HIPE foam. The emulsions used to prepare the HIPE foams can have water-to-oil phase ratios ranging from about 12:1 to 100:1; or from about 20:1 to 70:1; or also from about 25:1 to 50:1.

The continuous oil phase of the emulsions used to prepare HIPE foams comprises the monomers that will be polymerized to form the HIPE foam structure. Such monomers include a principal monomer component, a co-monomer component and a cross-linking agent component. Selection of particular types and amounts of principal monomer(s) and co-monomer(s) and poly-functional cross-linking agent(s) can be important to the realization of absorbent HIPE foams having the desired combination of structure, mechanical, and fluid handling properties, which render such HIPE foams suitable for use in the present invention.

According to an embodiment of the present invention, the substrate layer 100 of the absorbent core can comprise the layer of foam material 150. The layer of foam material 150 can typically constitute the substrate layer 100, as shown in Figure 3, or alternatively can be in contact with the substrate layer 100, for example being bonded to it with known means such as a construction adhesive, typically having its respective first surface bonded to the second surface of the substrate layer 100. In this latter case, typically the layer of foam material 150 can be coextensive with the substrate layer 100.

Certain foam materials provided as a layer with a selected thickness can comprise a backing layer, for example a fibrous layer such as typically a nonwoven layer. The foam material can be cast onto, or adhered to, or in any case combined with the fibrous layer, depending on the respective manufacturing process. A layer of foam material 150 of this type, i.e. for example in a composite structure with a nonwoven backing layer, in turn indicated as 150', is illustrated in the exemplary embodiment of Figure 3. This composite material can integrally constitute the substrate layer 100 of the absorbent core of the present invention, typically e.g. with the nonwoven backing layer 150' forming the first surface thereof, hence in turn in direct contact with the second surface of the layer of absorbent polymer material 110. The layer of foam material 150 can hence be provided as a composite structure where it is combined with a fibrous, e.g. a nonwoven, backing layer 150', or suitably combined with a fibrous substrate layer 100 in order to form a layered foam/fibre structure.

According to another embodiment of the present invention, illustrated in Figure 4, the cover layer 130 can comprise the layer of foam material 150, typically coextensive with it, or alternatively can be integrally constituted by the layer of foam material 150. Similarly to what has been explained above, a layer of foam material 150 combined with a nonwoven backing layer 150' can constitute the cover layer 130 wherein the nonwoven backing layer 150' can typically constitute the second surface thereof, in direct contact with the layer of adhesive 120.

According to the present invention, the layer of foam material 150 has a thickness from about 0.5 mm to about 3 mm, or also from about 1 mm to about 2 mm. Typically the thickness of the layer of foam material 150 can be substantially uniform. The foam material of the layer 150 can typically have a relatively low density, or, conversely, a relatively high bulkiness. Bulkiness, or bulk, of a material is meant herein as the inverse of its density, and can be typically expressed in cm³/g; it corresponds to the ratio of a given volume of the material, comprising pores or cells, as it occurs in a foam material, divided by its mass, wherein the volume is evaluated typically on the material in standard conditions, i.e. not subjected to any specific pressure, of course besides atmospheric pressure. Typically, the bulkiness is from about 20 cm³/g to about 200 cm³/g, or from about 50 cm³/g to about 150 cm³/g, or also from about 80 cm³/g to about 120 cm³/g. Thickness and bulkiness are referred to the foam material only, hence do not comprise for example any further layer combined with the layer of foam material 150, such as for example a backing layer 150'. According to an embodiment of the present invention, typically the bulkiness of the foam material can be substantially uniform throughout the layer of foam material 150.

The selection of the thickness of the layer of foam material 150 and/or of the bulkiness of the foam material as such comprised into the absorbent core of the present invention can provide for a better capability of the absorbent core, and in turn typically also of an absorbent article comprising it, e.g. a sanitary napkin, to withstand the forces and stresses experienced in use, and typically exerted by the wearer's body and by the undergarment, without permanently deforming and losing its original shape, particularly in the wet state, i.e. after absorption of body fluids. Particularly, the absorbent core of the present invention can be capable of a better degree of recovery after being compressed and folded on itself, for example typically along fold lines which in an absorbent article incorporating the absorbent core typically run longitudinally along the major axis of the product, as a result of mainly compressive forces acting generically within the plane of the absorbent article and perpendicularly to said longitudinal axis, as exerted for example by the wearer's thighs during wear of a sanitary napkin. The absorbent article with the absorbent core of the present invention is hence capable of better recovering from said deformed configuration, once the deforming forces are at least partially released. This can also typically translate into a better fluid acquisition and handling of the absorbent core and in turn of the absorbent article comprising it, as permanent deformations in use can be limited, with a reduced risk of bunching and/or roping, wherein the core and in turn the article can maintain a larger effective surface for liquid acquisition. Superior fit and comfort can hence be typically achieved without substantially reducing either the softness or the overall absorbency of the absorbent core and of the absorbent article, but instead with an even increased absorbent core utilization.

According to an embodiment of the present invention, the foam material can be hydrophilic, or also water absorbent. Affinity for water, either as hydrophilicity or actual absorption capacity, can provide the further advantage that the layer of foam material comprised in the absorbent core of the present invention, in addition to an improvement of its mechanical characteristics in response to stresses in use, as explained above, can also contribute to fluid acquisition and handling of the absorbent core structure, complementing the absorbent polymer material and the fibrous material which can be typically present in the absorbent core.

In the absorbent core of the present invention the substrate layer 100 and the optional cover layer 130, possibly in combination with the layer of foam material as explained above, can be typically provided from nonwoven materials, for example spunbonded or carded nonwoven materials, or also airlaid materials, such as for example latex and/or thermal bonded airlaid materials.

Exemplary materials for the substrate layer 100 can comprise fibrous materials comprising cellulose or cellulose derived fibres, typically not more than 90% by weight of cellulose or cellulose derived fibres, or from 40% to 80% by weight of cellulose or cellulose derived fibres. Examples of fibrous materials for the substrate layer 100 can be nonwoven materials, such as for example carded nonwovens, or more typically airlaid or wetlaid fibrous materials, such as for example latex or thermal bonded airlaid fibrous materials, comprising synthetic and natural fibres, such as for example cellulose fibres. Basis weights for the materials of the substrate layer 100 can typically range from 10 g/m² to 120 g/m², or from 40 g/m² to 100 g/m², or also from 50 g/m² to 80 g/m².

Exemplary materials for the optional cover layer 130 can be provided by nonwoven materials comprising synthetic fibres, such as polyethylene (PE), polyethylene terephthalate (PET), polypropylene (PP). As the polymers used for nonwoven production are inherently hydrophobic, they can be typically coated with hydrophilic coatings, for example with durably hydrophilic coatings to provide permanently hydrophilic nonwovens. Other nonwoven materials for the optional cover layer 130 can comprise composite structures such as a so called SMS material, comprising a spunbonded, a melt-blown and a further spunbonded layer. Basis weights for the materials of the cover layer 130 can typically range from 5 g/m² to 80 g/m², or from 10 g/m² to 60 g/m², or also from 20 g/m² to 40 g/m²

In certain embodiments of the present invention the absorbent polymer material 110 in the absorbent core 28 is present throughout the area of the absorbent core in an average basis weight of less than 250 g/m², or of less than 220 g/m², or from 60 g/m² to 180 g/m², or from 100g/m² to 160 g/m². An average basis weight is typically based on the whole area of the zone of application, i.e. interested by the layer of absorbent polymer material, and hence comprising possible openings included in an e.g. discontinuous layer. Typically, the absorbent polymer material 110 can constitute at least 45%, or at least 50%, or at least 55%, by weight of the absorbent core, wherein the absorbent core can typically correspond to the embodiments described with reference to Figures 3, 4, and 5, hence comprising the substrate layer, the layer of absorbent polymer material, the layer of thermoplastic material, the layer of foam material, the optional cover layer if present, and any other material possibly comprised within this structure, such as for example the additional fibrous material mentioned above or the additional adhesive material.

Typically the absorbent polymer material for the absorbent cores according to the present invention can comprise absorbent polymer particles typically having a selected average particle size. The absorbent polymer particles of the layer of absorbent polymer material 110 can typically have a selected average particle size from 200 µ to 600 µ, or from 300 µ to 500 µ.

According to the present invention, the absorbent core can provide a more efficient fluid management, in terms of acquisition, immobilization and absorption and a better comfort, during the entire wearing time of the article, as explained above, which can be particularly useful in case of complex body fluids such as menses or blood. Overall, this increased efficiency in the composite structure according to the present invention can translate in a more effective exploitation of the absorbent capacity of the absorbent polymer material, also in presence of problematic body fluids such as menses or blood or vaginal discharges, and possibly also in a more efficient use of the entire structure of the absorbent core.

This is achieved in a structure which is typically thin and flexible, yet capable of employing more completely the absorption and immobilization capacity of the different materials, and having improved fit and resilience during absorption and therefore increased comfort during use.

According to an embodiment of the present invention the absorbent polymer material can be selected among the polyacrylate based polymers described in the PCT Patent Application WO2007/047598, which are polyacrylate based materials very slightly crosslinked, or substantially not crosslinked at all, this further improving the above mentioned synergistic effect. Particularly, said polyacrylate based materials can have an extractable fraction of at least about 30% by weight, between 30% and 80% by weight, or between 32% and 70% by weight, evaluated according to the Extractables test method described in the above referenced application. Alternatively, said polyacrylate based materials can have a retention capacity of at least about 30 g/g, at least about 35 g/g, or at least about 40 g/g, evaluated according to the Centrifuge Retention Capacity test described in the above referenced application. The absorbent polymer material can also be selected among the polyacrylate based polymers described in the PCT Patent Application WO 07/046052. Said polymers in fact are particularly effective in absorbing complex body fluids such as menses or blood, and upon absorption of such fluids do not generally show a marked swelling, followed by gel blocking, like traditional superabsorbents, but rather act to a certain extent as thickeners of the body fluid, immobilizing it as a sort of gelatinous mass within the absorbent structure, for example in the interstices among the fibres, without causing substantial swelling and in turn a sensible increase of the overall thickness of the absorbent core.

According to the present invention, the absorbent core 28 can fully constitute the absorbent element in an absorbent article, or can be complemented with additional layers. Also, an absorbent article comprising an absorbent core according to the present invention can further comprise a fibrous acquisition layer between the absorbent core 28 and the topsheet. According to an embodiment of the present invention the acquisition layer can for example comprise fibrous nonwoven materials made by air laying or wet laying of synthetic fibres such as polyethylene (PE), polyethylene terephthalate (PET), or polypropylene (PP), similarly to the cover layer 130 of the absorbent core 28 of the present invention.

Exemplary materials for the fluid acquisition layer could comprise spunbonded or carded nonwoven materials, or airlaid materials such as for example latex bonded or thermal bonded airlaid materials. Basis weights can typically range from 10 g/m² to 60 g/m², or from 25 g/m² to 40 g/m².

According to another alternative embodiment of the present invention the absorbent article can comprise a further fibrous layer comprised between the absorbent core 28 and the backsheet, i.e. typically provided at the garment facing surface of the core. This optional layer can be provided by similar fibrous materials as those already described for the substrate layer 100 of the absorbent core of the present invention. This optional fibrous layer according to this further embodiment of the present invention can act as an added wicking layer receiving and distributing excess fluid which might not be fully retained by the absorbent core 28. The presence of cellulose fibres can make the layer particularly effective in acquiring and diffusing the fraction of body fluids like menses or blood which is not completely absorbed by the absorbent polymer material of the absorbent core 28.

The absorbent polymer material for the absorbent cores according to the present invention, typically comprising absorbent polymer particles, according to a further embodiment of the present invention, can have a permeability, as expressed by the saline flow conductivity of the absorbent polymer material, greater than 10, 20, 30 or 40 SFC- units, where 1 SFC unit is 1 x 10^{- 7}(cm³ x s) / g. Saline flow conductivity is a parameter well recognised in the art and is to be measured in accordance with the test disclosed in EP 752 892 B.

### Backsheet

The absorbent article of Figure 1 comprising the absorbent core according to the present invention can also comprise a backsheet 40. The backsheet may be used to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

### Test Methods

### Thickness of the layer of foam material and bulkiness of the foam material

Methods for the measurement of the thickness of a layer of foam material and of the bulkiness of the foam material itself are well known in the art.

The thickness of a layer of foam material can be suitably measured with methods which do not involve subjecting the sample material to any pressure, besides of course atmospheric pressure, and can typically be selected by the skilled person among optical methods, e.g. based on image analysis. Methods using Calibrated Image Analysis, or a Stage Micrometer are known and right at hand of the skilled person. The thickness of the foam layer may hence be accordingly measured on a cross section of the layer, for example on 25 mm x 25 mm square specimens cut from a layer of foam material, either from a standard sample of the material or from a sample taken from a commercial product. Length and width of the specimens shall also be accurately measured for example with a digital calliper.

Optical methods, such as those mentioned above, can easily allow the skilled person to measure the thickness of the foam layer as such, also when present within a composite material comprising the foam layer combined with a backing layer, e.g. a fibrous backing layer, or also in case the layer of foam material is taken from a commercial product, where for example it is bonded or in some way associated to another layer, for example a nonwoven layer, or material. In order to prepare the specimens the cut shall be clean and perpendicular to the plane of the specimen, taking care that the edge be not pinched together or distorted by the cut. The cut shall be done with a sharp razor blade, for example typically cut in guillotine fashion to get a neat edge. At least two thickness measurements shall be made on each side of the specimen, being equally spaced along the side; the thickness of each specimen is obtained as the average of all thickness measurements. Specimens shall be cut from at least three products or samples, and their respective thicknesses averaged. The resulting value shall be taken as the thickness of the foam layer.

The volume of each specimen is readily obtained from the respective thickness, length and width measurements.

In order to evaluate the bulkiness of the foam material, as explained expressed in cm³/g, the mass of the actual specimens used for the volume measures are then also measured. This is straightforward for a specimen only comprising a foam material. For specimens also comprising a backing layer, and in any case for specimens of foam material taken from commercial products where the foam material can be combined with other materials, e.g. a fibrous layer such as a nonwoven backing layer, the foam material shall be carefully and entirely separated from the other material or materials, e.g. a nonwoven layer and/or any adhesive material, and then weighed. Slight damages or irregularities on the surface of the foam material are not detrimental to the measurement, as the overall weight of the sample has to be measured. The bulkiness of each specimen shall be calculated from the respective volume and mass, and the average calculated from the individual results, which in turn is taken as the bulkiness of the foam material.

The tests shall be conducted at standard conditions of 23 ± 2°C and 50 ± 5% relative humidity, and the standard samples or the commercial products from which the specimens are to be taken shall be conditioned at the temperature and humidity of the test at least for 24 hours before being tested. Standard samples shall be conditioned in undeflected and undistorted state. In case of commercial products, they shall be taken out of any packaging or bag, and if necessary spread open. Specimens of the foam material shall be taken from commercial products in areas where the material is substantially flat, and does not comprise folds or creases.

### Rheological Creep Test

The Rheological Creep Test mentioned hereinabove for measuring the cohesive strength parameter y is as described in the copending patent application EP 1447067, assigned to the Procter & Gamble Company.

### Artificial Menstrual Fluid (AMF)

Artificial Menstrual Fluid is based on modified sheep's blood that has been modified to ensure it closely resembles human menstrual fluid in viscosity, electrical conductivity, surface tension and appearance. It is prepared as explained in US Patent 6,417,424, assigned to The Procter & Gamble Company, from line 33 of column 17 to line 45 of column 18, to which reference is made.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent core for an absorbent article intended for absorption of menses or blood or vaginal discharges, said core comprising a substrate layer,
said substrate layer comprising a first surface and a second surface,
said absorbent core further comprising a layer of absorbent polymer material,
said layer of absorbent polymer material comprising a first surface and a second surface, said absorbent core further comprising a layer of adhesive,
said layer of adhesive comprising a first surface and a second surface,
wherein said layer of absorbent polymer material is comprised between said layer of adhesive and said substrate layer;
said second surface of said layer of absorbent polymer material is in contact with said first surface of said substrate layer;
and said first surface of said layer of absorbent polymer material is in contact with said second surface of said layer of adhesive,
**characterized in that**
said absorbent core comprises a layer of foam material, said foam material being selected among melamine, polyurethane and high internal phase emulsions (HIPE) foams, and having a thickness from about 0.5 mm to about 3 mm, preferably from about 1 mm to about 2 mm and a bulkiness from about 20 cm³/g to about 200 cm³/g, preferably from about 50 cm³/g to about 150 cm³/g, more preferably from about 80 cm³/g to about 120 cm³/g.

2. An absorbent core according to any preceding claim, wherein said foam material is selected among melamine and polyurethane foams.

3. An absorbent core according to claim 1 or 2 further comprising a cover layer comprising a first surface and a second surface, wherein said second surface of said cover layer is in contact with said first surface of said layer of adhesive.

4. An absorbent core according to claims 1-3, wherein said substrate layer comprises said layer of foam material.

5. An absorbent core according to claim 1-2, wherein said substrate layer is constituted by said layer of foam material.

6. An absorbent core according to any preceding claim, wherein said foam material is hydrophilic.

7. An absorbent core according to any preceding claim, wherein said adhesive is fiberized.

8. An absorbent core according to any preceding claim, wherein said layer of absorbent polymer material is non uniform, and wherein said second surface of said non uniform layer of absorbent polymer material is in at least partial contact with said first surface of said substrate layer,
and portions of said second surface of said layer of adhesive are in contact with said first surface of said substrate layer and portions of said second surface of said layer of adhesive are in contact with said first surface of said non uniform layer of absorbent polymer material.

9. An absorbent article comprising a liquid permeable topsheet, a backsheet and an absorbent core according to any preceding claim comprised therebetween.

## Patentansprüche

1. Absorptionskern für einen Absorptionsartikel, der zur Absorption von Menstruationsflüssigkeit oder Blut oder Scheidenausfluss vorgesehen ist, wobei der Kern eine Substratschicht umfasst,
wobei die Substratschicht eine erste Oberfläche und eine zweite Oberfläche umfasst,
wobei der Absorptionskern ferner eine Schicht aus Polymerabsorptionsmaterial umfasst,
wobei die Schicht aus Polymerabsorptionsmaterial eine erste Oberfläche und eine zweite Oberfläche umfasst,
wobei der Absorptionskern ferner eine Haftmittelschicht umfasst,
wobei die Haftmittelschicht eine erste Oberfläche und eine zweite Oberfläche umfasst,
wobei die Schicht aus Polymerabsorptionsmaterial zwischen der Haftmittelschicht und der Substratschicht enthalten ist;
wobei die zweite Oberfläche der Schicht aus Polymerabsorptionsmaterial mit der ersten Oberfläche der Substratschicht in Kontakt steht;
und wobei die erste Oberfläche der Schicht aus Polymerabsorptionsmaterial mit der zweiten Oberfläche der Haftmittelschicht in Kontakt ist,
**dadurch gekennzeichnet, dass**
der Absorptionskern eine Schaummaterialschicht umfasst, wobei das Schaummaterial ausgewählt ist aus Melamin, Polyurethan und Schaumemulsionen mit hoher innerer Phase (HIPE), und mit einer Dicke von etwa 0,5 mm bis etwa 3 mm, vorzugsweise von etwa 1 mm bis etwa 2 mm und einer Bauschigkeit von etwa 20 cm³/g bis etwa 200 cm³/g, vorzugsweise von etwa 50 cm³/g bis etwa 150 cm³/g, besonders bevorzugt von etwa 80 cm³/g bis etwa 120 cm³/g.

2. Absorptionskern nach einem vorangehenden Anspruch, wobei das Schaummaterial ausgewählt ist aus Melamin- und Polyurethanschäumen.

3. Absorptionskern nach Anspruch 1 oder 2, ferner umfassend eine Deckschicht, die eine erste Oberfläche und eine zweite Oberfläche umfasst, wobei die zweite Oberfläche der Deckschicht in Kontakt mit der ersten Oberfläche der Haftmittelschicht steht.

4. Absorptionskern nach den Ansprüchen 1 bis 3, wobei die Substratschicht die Schaumstoffmaterialschicht umfasst.

5. Absorptionskern nach den Ansprüchen 1 bis 2, wobei die Substratschicht aus der Schaumstoffmaterialschicht gebildet ist.

6. Absorptionskern nach einem der vorstehenden Ansprüche, wobei das Schaummaterial hydrophil ist.

7. Absorptionskern nach einem der vorstehenden Ansprüche, wobei das Haftmittel zerfasert ist.

8. Absorptionskern nach einem der vorstehenden Ansprüche, wobei die Schicht aus Polymerabsorptionsmaterial ungleichmäßig ist, und wobei die zweite Oberfläche der ungleichmäßigen Schicht aus Polymerabsorptionsmaterial in mindestens teilweisem Kontakt mit der ersten Oberfläche der Substratschicht ist,
und wobei Abschnitte der zweiten Oberfläche der Haftmittelschicht mit der ersten Oberfläche der Substratschicht in Kontakt stehen und Abschnitte der zweiten Oberfläche der Haftmittelschicht mit der ersten Oberfläche der ungleichmäßigen Schicht aus Polymerabsorptionsmaterial in Kontakt stehen.

9. Absorptionskern, der eine flüssigkeitsdurchlässige Oberschicht, eine Unterschicht und einen Absorptionskern nach einem der vorstehenden Ansprüche, der dazwischen enthalten ist, umfasst.

## Revendications

1. Âme absorbante pour un article absorbant prévu pour l'absorption des règles ou de sang ou d'écoulements vaginaux, ladite âme comprenant une couche de substrat,
ladite couche de substrat comprenant une première surface et une deuxième surface,
ladite âme absorbante comprenant en outre une couche de matériau polymère absorbant,
ladite couche de matériau polymère absorbant comprenant une première surface et une deuxième surface,
ladite âme absorbante comprenant en outre une couche d'adhésif,
ladite couche d'adhésif comprenant une première surface et une deuxième surface,
dans laquelle ladite couche de matériau polymère absorbant est comprise entre ladite couche d'adhésif et ladite couche de substrat ;
ladite deuxième surface de ladite couche de matériau polymère absorbant est en contact avec ladite première surface de ladite couche de substrat ;
et ladite première surface de ladite couche de matériau polymère absorbant est en contact avec ladite deuxième surface de ladite couche d'adhésif,
**caractérisée en ce que**
ladite âme absorbante comprend une couche de matériau en mousse, ledit matériau en mousse étant choisi parmi des mousses de mélamine, de polyuréthane et d'émulsions à phase interne élevée (HIPE), et ayant une épaisseur d'environ 0,5 mm à environ 3 mm, de préférence d'environ 1 mm à environ 2 mm et un encombrement d'environ 20 cm³/g à environ 200 cm³/g, de préférence d'environ 50 cm³/g à environ 150 cm³/g, plus préférablement d'environ 80 cm³/g à environ 120 cm³/g.

2. Âme absorbante selon une quelconque revendication précédente, dans laquelle ledit matériau en mousse est choisi parmi des mousses de mélamine et de polyuréthane.

3. Âme absorbante selon la revendication 1 ou 2 comprenant en outre une couche de couverture comprenant une première surface et une deuxième surface, dans laquelle ladite deuxième surface de ladite couche de couverture est en contact avec ladite première surface de ladite couche d'adhésif.

4. Âme absorbante selon les revendications 1 à 3, dans laquelle ladite couche de substrat comprend ladite couche de matériau en mousse.

5. Âme absorbante selon la revendication 1 ou 2, dans laquelle ladite couche de substrat est constituée de ladite couche de matériau en mousse.

6. Âme absorbante selon une quelconque revendication précédente, dans laquelle ledit matériau en mousse est hydrophile.

7. Âme absorbante selon une quelconque revendication précédente, dans laquelle ledit adhésif est rendu fibreux.

8. Âme absorbante selon une quelconque revendication précédente, dans laquelle ladite couche de matériau polymère absorbant est non uniforme, et dans laquelle ladite deuxième surface de ladite couche non uniforme de matériau polymère absorbant est en contact au moins partiel avec ladite première surface de ladite couche de substrat,
et des parties de ladite deuxième surface de ladite couche d'adhésif sont en contact avec ladite première surface de ladite couche de substrat et des parties de ladite deuxième surface de ladite couche d'adhésif sont en contact avec ladite première surface de ladite couche non uniforme de matériau polymère absorbant.

9. Article absorbant comprenant une feuille de dessus perméable aux liquides, une feuille de fond et une âme absorbante selon une quelconque revendication précédente comprise entre elles.
